# EUROPEAN PATENT APPLICATION

(11) **EP 3 293 187 A1**
(43) Date of publication of application: **14.03.2018**
(21) Application number: 17189823.2
(22) Date of filing: 07.09.2017
(51) Int. Cl.: C07D 495/04

(54) **A PROCESS FOR THE PREPARATION OF A SUBSTITUTED IMIDAZOTHIAZOLONE COMPOUND**

(30) Priority: 07.09.2016 WO PCT/CN2016/098302
(71) Applicant: DSM IP Assets B.V., 6411 TE Heerlen (NL)
(72) Inventor: ZHU, Zhibin, 4303 Kaiseraugst (CH); Zhang, Lei, 4303 Kaiseraugst (CH); PENG, Kun, 4303 Kaiseraugst (CH); BONRATH, Werner, 4303 Kaiseraugst (CH)
(74) Representative: Kurt, Manfred

(57) **Abstract**

The present invention provides a new process for the preparation of a substituted imidazothiazolone compound. The process of the present invention uses a fluoride-free Lewis acid which is cheap and friendly to environment, and provide high selectivity and yield.

## Description

### Field of the invention

The present invention is related to a process for the preparation of a substituted imidazothiazolone compound, a very useful intermediate for (+)-biotin.

### Background of the invention

Biotin (Vitamin H) is one of the B-complex group of vitamins and has immense commercial importance in the area of animal health and nutrition. It is one of the biocatalysts of the reversible metabolic reactions of carbon dioxide transport in micro and macro organisms. It is used in poultry feeds of rapid growth of chicks and healthy hatching of eggs. Biotin avidin complex finds a vital role in the area of biochemistry.

To date, a number of synthetic routes of biotin have been reported. Of the various approaches described toward (+)-biotin synthesis, lactone-thiolactone process developed by Hoffman-La Roche represents the best commercial advantage (see: Sternbach, L. H. Comp. Biochem. 1963, 11, 66).

In recent years, cysteine has attracted a great deal of attention by virtue of it possessing requisite stereochemistry and its ready availability. A bicyclic hydantoin can derived from L-cysteine and then be reduced to afford a hydroxyl imidazothiazolone, which is treated with nucleophile in the presence of a Lewis acid to afford a substituted imidazothiazolone, a very important intermediate which can be converted to (+)-biotin. (see: US patent publication No.5,274,107; and Subhash P. Chavan, et al., J. Org. Chem., 2005, 70, 1901-1903)

All these processes are however characterized by large number of synthetic steps resulting in low overall yields or involve highly toxic, expensive and hazardous chemicals, for example, carbonyldiimidazole, methyl Iodide, potassium cyanide and BF₃•OEt₂ *etc.* which are not good for health or environment.

The object of the present invention is to provide a substituted imidazothiazolone which can be used for synthesis of (+)-biotin.

### Summary of the invention

The present invention provides a new process for the preparation of a substituted imidazothiazolone compound of formula (I), or a stereoisomer thereof, or a stereoisomeric mixture thereof, which comprises:
reacting a compound of formula (II), or a stereoisomer thereof, or a stereoisomeric mixture thereof, with a nucleophile in the presence of a fluoride-free Lewis acid to provide the compound of formula (I), or a stereoisomer thereof, or a stereoisomeric mixture thereof: 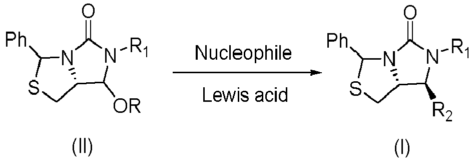
wherein: R is H, alkyl or alkylcarbonyl; R₁ is benzyl; and R₂ is alkyl group, preferably, 1-phenyl-1-ethanonyl, 1-(4-chlorophenyl)-1-ethanonyl, 1-(4-methoxypheny)-1-ethanonyl, 2-oxocyclohexyl, 1-trimethylsilyloxy-2-oxocyclohexyl, 1-hydroxyl-2-oxocyclohexyl, or 2-methylpropanoate.

The process of the present invention uses a fluoride-free Lewis acid which is cheap and friendly to environment, and provide high selectivity and yield.

### Detailed description of the invention

In the present invention, the term "alkyl" refers to unsubstituted or substituted straight- or branched-chain hydrocarbon groups having 1-20 carbon atoms, preferably 1-7 carbon atoms. Exemplary unsubstituted alkyl groups include methyl, ethyl, propyl, isopropyl, n-butyl, t-butyl, isobutyl, pentyl, neopentyl, hexyl, isohexyl, heptyl, octyl and the like.

In the present invention, the term "alkylcarbonyl" refer to the structure "alkyl-CO-" wherein alkyl is defined as above.

The present invention provides a new process for the preparation of a substituted imidazothiazolone compound of formula (I), or a stereoisomer thereof, or a stereoisomeric mixture thereof, which comprises:
reacting a compound of formula (II), or a stereoisomer thereof, or a stereoisomeric mixture thereof, with a nucleophile in the presence of a fluoride-free Lewis acid to provide the compound of formula (I), or a stereoisomer thereof, or a stereoisomeric mixture thereof: 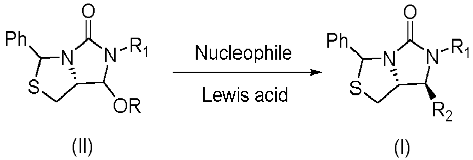
wherein: R is H, alkyl or alkylcarbonyl; R₁ is benzyl; and R₂ is alkyl group, preferably, 1-phenyl-1-ethanonyl, 1-(4-chlorophenyl)-1-ethanonyl, 1-(4-methoxypheny)-1-ethanonyl, 2-oxocyclohexyl, 1-trimethylsilyloxy-2-oxocyclohexyl, 1-hydroxyl-2-oxocyclohexyl, or 2-methylpropanoate.

| R₂ | Chemical structures |
|---|---|
| 1-phenyl-1-ethanonyl | 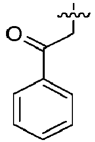 |
| 1-(4-chlorophenyl)-1-ethanonyl | 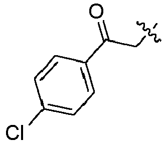 |
| 1-(4-methoxypheny)-1-ethanonyl | 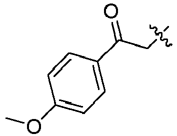 |
| 2-oxocyclohexyl | 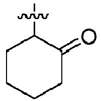 |
| 1-trimethylsilyloxy-2-oxocyclohexyl | 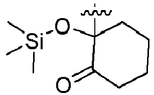 |
| 1-hydroxyl-2-oxocyclohexyl | 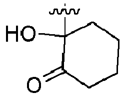 |
| 2-methylpropanoate | 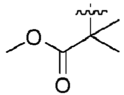 |

The stereoisomer of the present invention includes enantiomers and diastereomers. For example, the compound of the formula (I) has the following stereoisomers: 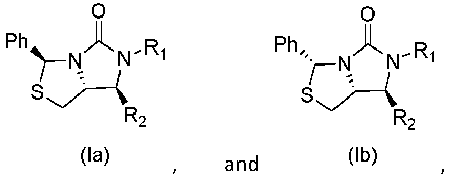 and the compound of the formula (II) has the following stereoisomers:

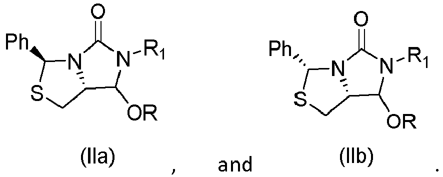

In the process of the present invention, the nucleophile is selected from enol ethers such as silyl enol ethers. Preferably, the nucleophile suitable for the process of the present invention is selected from the group consisting of the following structures (a)-(f):

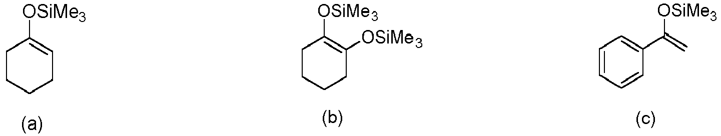

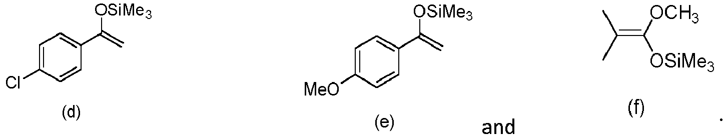

The amount of the nucleophile used in the process of the present invention may be 0.5-8 eq., preferably 0.8-4 eq., more preferably 1-3 eq., based on the amount of the compound of formula (II).

In the process of the present invention, the fluoride-free Lewis acid is zinc halide such as ZnCl₂, ZnBr₂, and Znl₂. Preferably, the fluoride-free Lewis acid is ZnCl₂.

The amount of the fluoride-free Lewis acid used in the process of the present invention may be 0.01-4 eq., preferably 0.05-2.0 eq., more preferably 0.1-1.5 eq., based on the amount of the compound of formula (II).

Preferably, an organic solvent may be used in the process of the present invention. The organic solvent used may be selected from the group consisting of ether such as tetrahydrofuran, hydrocarbons such as benzene and toluene, and chlorinated hydrocarbons such as chloroform, dichloromethane (DCM) and dichloroethane.

The process of the present invention may be carried out at a temperature of from -10°C to 30°C, preferably, from 0°C to room temperature.

The compound of the formula (II) may be prepared by known procedures, for example, as disclosed in Ke-Xi CHEN et al., Chinese Journal of Organic Chemistry, Vol. 26, 2006, No. 9, 1309-1312.

The compound of the formula (I) may be used for the preparation of (+)-biotin by known procedures, for example, as disclosed in Ke-Xi CHEN et al., Chinese Journal of Organic Chemistry, Vol. 26, 2006, No. 9, 1309-1312.

The merits of the process of the present invention are use of cheap, non-toxic and a fluoride free Lewis acid, and of high yield and selectivity.

The following Examples are intended to further illustrate the invention and are not to be construed as being limitations thereon.

### Examples

### Example 1: (3S,7R,7aR)-6-benzyl-7-((R)-1-hydroxy-2-oxocyclohexyl)-3-phenyltetrahydro-3H,5H-imidazo[1,5-c]thiazol-5-one (compound 3)

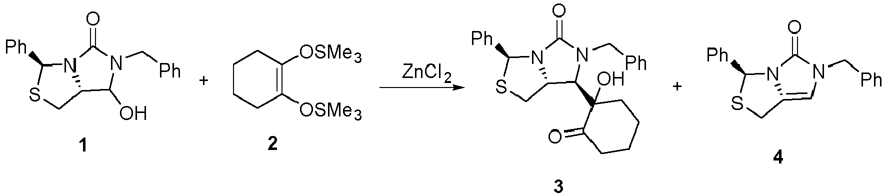

In a 50 mL Schlenk tube, the compound **1** (0.5 g, 1.451 mmol) and ZnCl₂ (0.222 g, 1.596 mmol) in DCM (9.94 ml) were added to give a white suspension. The compound **2** (0.789 g, 2.90 mmol) was added at 0°C and stirred for further 1h at room temperature.

The solvent was removed under vacuum and the reaction mixture was diluted with MeOH (9.94 ml), and sodium hydroxide (0.290 g, 7.25 mmol) were added and stirred for 1 h.

The solvent was removed and water 10 mL was added. The mixture was extracted with DCM (10 mL) for three times, dried with Na₂SO₄ and then the solvent was removed under vacuum, to obtain the compound **3** (0.56 g) and the compound **4** (4.4 mg) with the following data in Table 1.

**Table 1**

| Lewis Acid (eq.) | T (°C) | Yield (%) | | Selectivity (%) |
|---|---|---|---|---|
| | | Compound **3** | Compound **4** | |
| ZnCl₂ (1.1) | 0 | 91.0 | 1.0 | 99 |

### Example 2: Preparation of compound 3 by using other Lewis acids

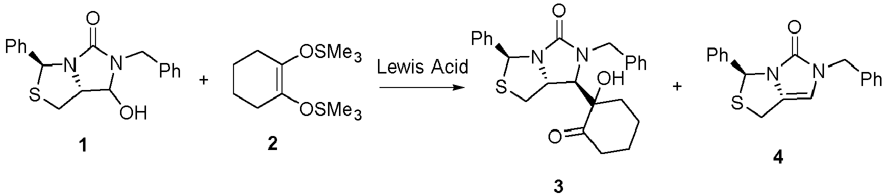

The compound **3** and the compound **4** were obtained according to the same process of Example 1 with the following conditions. The results were tested with LC-MS as indicated in Table 2.

**Table 2**

| No. | Amount of compound **1** | Amount of compound **2** | Lewis Acid (eq.) | T (°C) | Selectivity of compound **3** (%) |
|---|---|---|---|---|---|
| 1 | 6.0 g | 9.5 g | BF₃•OEt₂ (1.1) | 0 | 90 |
| 2* | 0.5 g | 0.8 g | FeCl₃ (1.1) | 0-25 | 91 |
| 3 | 0.5 g | 0.8 g | Ac₂O (1.5) | 0-25 | - |
| 4 | 0.5 g | 0.8 g | AcOH (1.5) | 0-25 | - |
| 5 | 0.5 g | 0.8 g | ZnBr₂ (1.1) | 0-25 | 83 |
| 6 | 0.5 g | 0.8 g | Zn(OTf)₂ (1.1) | 0-25 | 83 |
| 7 | 0.5 g | 0.8 g | TsOH (1.1) | 0-25 | 17 |
| 8 | 0.5 g | 0.8 g | TFA (0.5) | 0-25 | - |
| 9 | 0.5 g | 0.8 g | ZnCl₂(0.1) | 0-25 | 92% |

| | | | | | |
|---|---|---|---|---|---|
| * NaOH treatment was not necessary in this entry. | | | | | |

## Claims

1. A process for the preparation of a substituted imidazothiazolone compound of formula (I), or a stereoisomer thereof, or a stereoisomeric mixture thereof, which comprises:
reacting a compound of formula (II), or a stereoisomer thereof, or a stereoisomeric mixture thereof, with a nucleophile in the presence of a fluoride-free Lewis acid to provide the compound of formula (I), or a stereoisomer thereof, or a stereoisomeric mixture thereof: 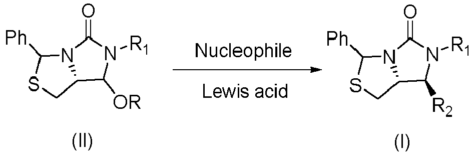
wherein: R is H, alkyl or alkylcarbonyl; R₁ is benzyl; and R₂ is alkyl group,

2. The process of claim 1, wherein R₂ is 1-phenyl-1-ethanonyl, 1-(4-chlorophenyl)-1-ethanonyl, 1-(4-methoxypheny)-1-ethanonyl, 2-oxocyclohexyl, 1-trimethylsilyloxy-2-oxocyclohexyl, 1-hydroxyl-2-oxocyclohexyl, or 2-methylpropanoate.

3. The process of claim 1 or 2, wherein the nucleophile is selected from enol ethers such as silyl enol ethers.

4. The process of claim 1 or 2, wherein the nucleophile is selected from the group consisting of the following structures (a)-(f):

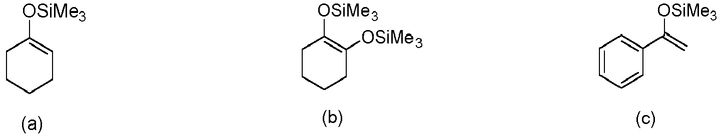

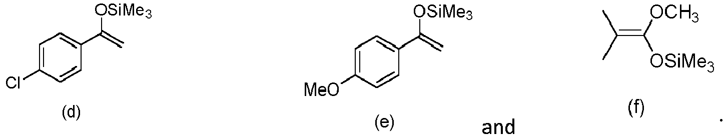

5. The process of claim 1 or 2, wherein the fluoride-free Lewis acid is zinc halide such as ZinCl₂, ZnBr₂, and Znl₂.

6. The process of any one of claims 1-5, wherein an organic solvent is used.

7. The process of claim 6, wherein the organic solvent used may be selected from the group consisting of ether such as tetrahydrofuran, hydrocarbons such as benzene and toluene, and chlorinated hydrocarbons such as chloroform, dichloromethane and dichloroethane.

8. The process of any one of claims 1-7, wherein the reaction is carried out at a temperature of from -10°C to 30°C, preferably, from 0°C to room temperature.
